# FASCICULE DE BREVET EUROPEEN

(11) **EP 0 486 385 B1**
(45) Date de publication et mention de la délivrance du brevet: **28.12.1994**
(21) Numéro de dépôt: 91403043.2
(22) Date de dépôt: 14.11.1991
(51) Int. Cl.: C07D 233/64, A61K 31/415, C07C 53/46, C07C 57/76, C07C 49/16, C07C 49/163, C07C 49/213, C07C 49/215, C07D 401/06

(54) **Dérivés d'imidazole, leur procédé de préparation et les compositions pharmaceutiques qui les contiennent**
Imidazol-Derivate, Verfahren zu deren Herstellung und diese enthaltende Zusammenstellungen
Imidazole derivatives, process for their fabrication and pharmaceutical compositions containing them

(30) Priorité: 14.11.1990 FR 9014086
(43) Date de publication de la demande: 20.05.1992
(73) Titulaire: ADIR ET COMPAGNIE, 92415 Courbevoie Cédex (FR)
(72) Inventeur: Malen, Charles, F-94260 Fresnes (FR); de Nanteuil, Guillaume, F-92150 Suresnes (FR)

(56) Documents cités:
- EP-A- 0 247 764
- US-A- 4 497 818

## Description

La présente invention concerne de nouveaux dérivés d'imidazole, leur procédé de préparation et les compositions pharmaceutiques qui les contiennent.

De très nombreux dérivés de l'imidazole ont été décrits dans la littérature. Certains d'entre eux interagissent plus spécifiquement avec les récepteurs α₂ adrénergiques et présentent des propriétés sédatives pour les agonistes, antidépressantes pour les antagonistes.

Les brevets EP 247 764, EP 183 492 et US 44 97818, par exemple, décrivent des composés qui sont des antagonistes α₂ adrénergiques.

Les composés de la présente invention se différencient de ceux décrits dans l'Art antérieur par le fait que l'imidazole est substitué en position 4(5) par une chaîne aliphatique comportant un groupement trifluorométhyle.

D'autre part, outre le fait qu'ils soient nouveaux, ils possèdent des propriétés pharmacologiques très intéressantes.

En effet leur action s'exerce tant au niveau du système nerveux central qu'au niveau du système nerveux périphérique par le biais des récepteurs α₂ pour lesquels ils présentent une très forte affinité accompagnée d'une très grande sélectivité par rapport aux récepteurs α₁ adrénergiques.

De plus, de par leur action antagoniste α₂, ils facilitent la libération de noradrénaline à partir des terminaisons sympathiques et peuvent donc être utilisés dans le traitement des maladies qui sont liées à une déficience en noradrénaline disponible sur les récepteurs adrénergiques postsynaptiques du système nerveux central ou périphérique comme la dépression, l'hypertension, la maladie de Parkinson, l'asthme, les états convulsivants ou l'aggrégation plaquettaire.

Enfin, le métabolisme des glucides et des lipides étant régulé par un mécanisme d'inhibition au niveau des récepteurs α₂, les composés de la présente invention sont également utiles dans le traitement des maladies métaboliques comme le diabète et l'obésité.

La présente invention concerne plus particulièrement les dérivés de formule (I) : 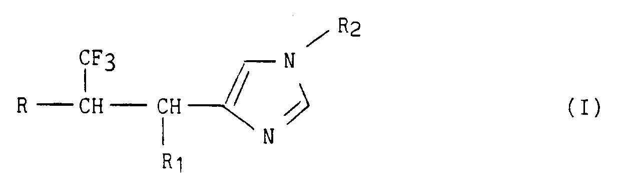
dans laquelle
R représente un groupement alkyle inférieur (C₁-C₆) linéaire ou ramifié, un groupement cycloalkyle (C₃-C₈), un groupement phényle (éventuellement substitué par un ou plusieurs atomes d'halogène, un ou plusieurs groupements alkyles (C₁-C₆) linéaires ou ramifiés eux-mêmes éventuellement substitués par un ou plusieurs atomes d'halogène, un ou plusieurs groupements alkoxy (C₁-C₆) linéaires ou ramifiés), un groupement pyridyle, un groupement naphtyl,
R identiques ou différents représentent un atome d'hydrogène, un groupement alkyle (C₁-C₆) linéaire ou ramifié, un groupement acyle (C₁-C₆) linéaire ou ramifié,
leurs énantiomères, diastéréoisomères et épimères ainsi que leurs sels d'addition à un acide pharmaceutiquement acceptable.

Parmi les acides pharmaceutiquement acceptables, on peut citer à titre non limitatif les acides chlorhydrique, sulfurique, tartrique, maléïque, fumarique, oxalique, méthanesulfonique, camphorique, etc...

L'invention s'étend aussi au procédé de préparation des composés de formule (I) qui peut être caractérisé en ce que l'on soumet un composé organomagnésien de formule (II)

R - MgX (II)

dans laquelle
R a la même signification que précédemment et X représente un atome d'halogène
à l'action de l'acide trifluoroacétique ou de son sel de sodium,
pour conduire à une cétone de formule (III) 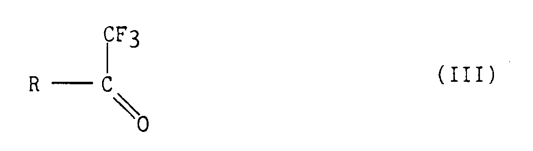
dans laquelle R a la même signification que dans la formule (I),
que l'on fait réagir avec les phosphonoacétates de formule (IV), en présence d'une base, selon la réaction de Horner-Emmons, 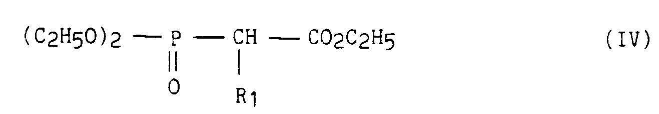
dans laquelle R₁ a la même signification que dans la formule (I),
pour conduire aux esters insaturés de formule (V) 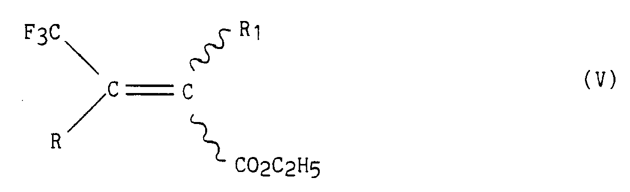
dans laquelle R et R₁ ont la même signification que dans la formule (I),
que l'on soumet à une hydrogénation catalytique en utilisant, par exemple,
comme catalyseur du charbon palladié ou de l'oxyde de platine,
pour conduire aux esters de formule (VI) 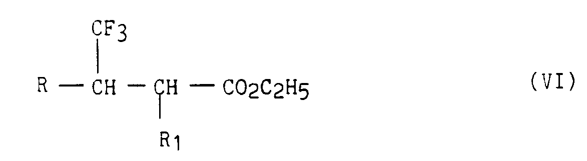
dans laquelle R et R₁ ont la même signification que dans la formule (I),
que l'on saponifie en présence de potasse ou de soude aqueuse,
pour conduire aux acides correspondants de formule (VII) 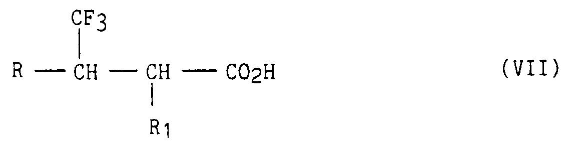
dans laquelle R et R₁ ont la même signification que dans la formule (I),
que l'on soumet à l'action du chlorure de thionyle,
pour conduire aux chlorures d'acide correspondants de formule (VIII) 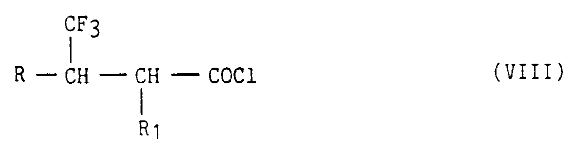
dans laquelle R et R₁ ont la même signification que dans la formule (I),
que l'on traite ensuite avec un excès de diazométhane,
pour conduire aux diazocétones de formule (IX) 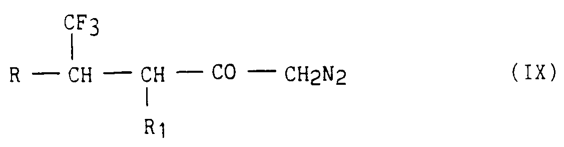
dans laquelle R et R₁ ont la même signification que dans la formule (I),
que l'on soumet à l'action de l'acide chlorhydrique gazeux,
pour conduire aux chlorocétones correspondantes de formule (X) 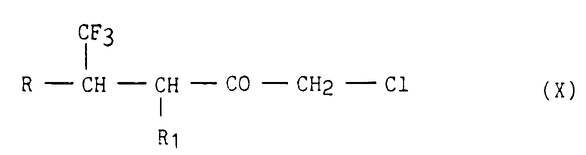
dans laquelle R et R₁ ont la même signification que dans la formule (I) que l'on fait réagir avec l'acétate de formamidine dans un solvant à haut point d'ébullition comme par exemple le diéthylène glycol,
pour conduire aux dérivés de formule (I),
que l'on purifie par des techniques classiques de purification, dont on sépare éventuellement les isomères selon les techniques classiques de séparation et que l'on transforme, si on le désire, en leurs sels d'addition à un acide pharmaceutiquement acceptable.

Les composés de formule (VIII), (IX) et (X) sont nouveaux et font partie de l'invention au même titre que les composés de formule (I) dont ils constituent les intermédiaires de synthèse.

La présente invention a également pour objet les compositions pharmaceutiques renfermant comme principe actif au moins un composé de formule générale (I) ou un de ses sels d'addition à un acide pharmaceutiquement acceptable, seul ou en combinaison avec un ou plusieurs excipients ou véhicules inertes, non toxiques.

Parmi les compositions pharmaceutiques selon l'invention on pourra citer plus particulièrement celles qui conviennent pour l'administration orale, parentérale, nasale, rectale, les comprimés simples ou dragéifiés, les comprimés sublinguaux, les sachets, les paquets, les gélules, les glossettes, les tablettes, les suppositoires, les crèmes, pommades, gels dermiques, etc...

La posologie utile varie selon l'âge et le poids du patient, la nature et la sévérité de l'affection ainsi que la voie d'administration. Celle-ci peut être orale, nasale, rectale ou parentérale. D'une manière générale, la posologie unitaire s'échelonne entre 0,1 et 100 mg pour un traitement en 1 à 3 prises par 24 heures.

Les exemples suivants illustrent l'invention et ne la limitent en aucune façon.

### EXEMPLE 1 : 4-(2-Cyclopentyl-3,3,3-trifluoropropan-1-yl) imidazole, chlorhydrate

### STADE A : Cyclopentyltrifluorométhylcétone

Une suspension de trifluoroacétate de sodium, préparée en additionnant lentement, à 0°C, 0,5 mole d'acide trifluoroacétique en solution dans le THF à 0,5 mole d'hydrure de sodium en suspension dans le THF, est ajoutée à un réactif organomagnésien lui-même préparé par addition, goutte à goutte, en entretenant le reflux, de 0,5 mole de bromure de cyclopentyle en solution dans 400 ml d'éther éthylique sur 13,4 g de tournures de magnésium placées dans 50 ml d'éther éthylique anhydre avec un cristal d'iode, le reflux étant maintenu une heure.

La suspension blanche ainsi obtenue est portée à reflux 2 heures 30 et maintenue à température ambiante une nuit.

Le mélange est alors hydrolysé, à 0°C, par 200 ml d'acide chlorhydrique 6N et 250 ml d'eau.

La phase organique est alors lavée par de l'eau, puis par une solution bicarbonatée à 10 % et à nouveau par de l'eau. Après séchage et évaporation des solvants, le produit attendu est obtenu par distillation.
Point d'ébullition : 60-65°C (150 mm/Hg)
N_{D}^{21,5°C}= 1,377

### STADE B : 3-Cyclopentyl-4,4,4-trifluoro-2-buténoate d'éthyle

A 120 mmoles d'hydrure de sodium en suspension dans 110 ml de diméthoxyéthane on ajoute, goutte à goutte, à température ambiante, 120 mmoles de triéthylphosphonoacétate en solution dans 50 ml de diméthoxyéthane, en maintenant une température inférieure à 35°C. Ce mélange est alors laissé 24 heures sous agitation à température ambiante.

120 mmoles du produit obtenu au stade précédent sont alors additionnées goutte à goutte.

Après une heure d'agitation à température ambiante, le mélange est versé sur un litre d'eau. Après extraction par de l'éther, lavage des phases éthérées par de l'eau, séchage et évaporation, le produit attendu est obtenu par distillation sous vide.
Rendement : 88 %
Point d'ébullition : 105°C (30 mm/Hg)
N_{D}^{20,5°C}= 1,428

| Microanalyse élémentaire : | | |
|---|---|---|
| | C % | H % |
| calculé | 55,93 | 6,40 |
| trouvé | 55,69 | 6,37 |

### STADE C : 3-Cyclopentyl-4,4,4-trifluorobutanoate d'éthyle

97 mmoles du produit obtenu au stade précédent sont hydrogénées à température et pression ambiantes dans 200 ml de méthanol en présence de 1 g de Palladium sur charbon à 10 %.

Après une nuit, le produit attendu est obtenu sous forme d'huile incolore, après filtration du catalyseur et évaporation du solvant.
Rendement : 98 %
N_{D}^{20,5°C}= 1,412

| Microanalyse élémentaire : | | |
|---|---|---|
| | C % | H % |
| calculé | 55,45 | 7,19 |
| trouvé | 55,21 | 7,01 |

### STADE D : Acide 3-cyclopentyl-4,4,4-trifluorobutanoïque

A 86 mmoles du produit obtenu au stade précédent dans 35 ml de méthanol, on ajoute sous refroidissement de glace, 170 mmoles de potasse aqueuse 2N.

Après une nuit à température ambiante et 2 heures 30 de reflux, le méthanol est évaporé. Le résidu est alors extrait par de l'éther, la phase aqueuse acidifiée par de l'acide chlorhydrique 1N est à nouveau extraite par de l'éther.

Le produit attendu est obtenu après séchage et évaporation des phases éthérées, sous forme d'huile incolore.
Rendement : 97 %
N_{D}^{22,5°C}= 1,419

### STADE E : 1-Chloro-4-cyclopentyle-5,5,5-trifluoropentan-2-one

86 mmoles du produit obtenu au stade précédent placées avec 430 mmoles de chlorure de thionyle sont portées à reflux pendant 1 heure 30.

Le chlorure d'acide correspondant est obtenu après évaporation du chlorure de thionyle en excès. Il est alors additionné sur 170 mmoles de diazométhane dans 800 ml d'éther éthylique. L'ensemble est abandonné 1 heure à -5°C puis 1 heure à +5°C. Un barbottage d'acide chlorhydrique gazeux et sec est ensuite réalisé dans cette solution, à 0°C, pendant 45 minutes. L'ensemble est laissé une nuit en glacière. Après addition de 100 ml d'eau, la phase éthérée est extraite, lavée par une solution de bicarbonate de sodium à 10 %, séchée et évaporée.

Le produit attendu est alors obtenu par distillation sous vide.
Rendement : 78 %
Point d'ébullition : 60-65°C (0,01 mm/Hg)

| Microanalyse élémentaire : | | | |
|---|---|---|---|
| | C % | H % | Cl % |
| calculé | 49,50 | 5,82 | 14,61 |
| trouvé | 49,30 | 5,75 | 14,80 |

### STADE F : 4-(2-Cyclopentyl-3,3,3-trifluoropropan-1-yl) imidazole, chlorhydrate

8 mmoles du produit obtenu au stade précédent sont placées dans 50 ml de diéthylène glycol avec 32 mmoles d'acétate de formamidine. L'ensemble est chauffé 50 heures à 135°C.

Après refroidissement, 70 ml d'éther et 30 ml d'eau sont additionnés au mélange précédent. La phase aqueuse est extraite plusieurs fois par de l'éther. Les phases éthérées sont réunies, séchées puis évaporées.

Le produit attendu est obtenu, sous forme de base, après purification par chromatographie sur silice en utilisant comme solvant d'élution un mélange heptane/éthanol/ammoniaque (100/5/0,05). Le chlorhydrate est obtenu au moyen d'éther chlorhydrique et est recristallisé dans un mélange acétate d'éthyle-éther isopropylique.
Rendement : 34 %
Point de fusion : 99-100°C

| Microanalyse élémentaire : | | | | |
|---|---|---|---|---|
| | C % | H % | N % | Cl % |
| calculé | 49,17 | 6,00 | 10,43 | 13,19 |
| trouvé | 49,40 | 6,05 | 10,54 | 13,16 |

### EXEMPLE 2 : 4-(2-Phényl-3,3,3-trifluoropropan-1-yl) imidazole, chlorhydrate

Le produit attendu est obtenu en procédant comme dans l'exemple 1 (stades B à F), mais en remplaçant au stade B la cyclopentyltrifluorométhylcétone par la phényltrifluorométhylcétone et en réalisant au stade C une hydrogénation catalytique en présence d'oxyde de platine.
Point de fusion : 202-205°C

| Microanalyse élémentaire : | | | | |
|---|---|---|---|---|
| | C % | H % | N % | Cl % |
| calculé | 52,09 | 4,37 | 10,12 | 12,81 |
| trouvé | 52,13 | 4,45 | 10,12 | 12,69 |

### EXEMPLE 3 : 4-(2-Cyclohexyl-3,3,3-trifluoropropan-1-yl) imidazole, chlorhydrate

Le produit attendu est obtenu en procédant comme dans l'exemple 1 mais en remplaçant au stade A le bromure de cyclopentyle par le bromure de cyclohexyle.
Point de fusion : 145-150°C

| Microanalyse élémentaire : | | | | |
|---|---|---|---|---|
| | C % | H % | N % | Cl % |
| calculé | 50,98 | 6,42 | 9,91 | 12,54 |
| trouvé | 51,12 | 6,20 | 9,80 | 12,63 |

### EXEMPLE 4 : 4-(2-Cycloheptyl-3,3,3-trifluoropropan-1-yl) imidazole

Le produit attendu est obtenu en procédant comme dans l'exemple 1 mais en remplaçant au stade A, le bromure de cyclopentyle par le bromure de cycloheptyle.
Point de fusion : 185-190°C

| Microanalyse élémentaire : | | | | |
|---|---|---|---|---|
| | C % | H % | N % | Cl % |
| calculé | 52,62 | 6,79 | 9,44 | 11,95 |
| trouvé | 52,66 | 6,83 | 9,25 | 11,93 |

### ETUDE PHARMACOLOGIQUE DES DERIVES DE L'INVENTION

### EXEMPLE 5 : Test d'affinité in vitro pour les récepteurs α₂ et α₁

Les tests d'affinité in vitro pour les récepteurs α₂ et α₁ ont été réalisés selon des techniques classiques de binding. Les préparations membranaires utilisées sont des microsomes de système nerveux central de rat.

Les résultats de ces études montrent, par exemple, que le composé de l'exemple 2 possède un K_{0,5} de l'ordre de 5.10⁻⁸ (mole/litre, n=1) vis à vis des récepteurs α₂ et supérieur à 10⁻⁴ vis à vis des récepteurs α₁.

Le rapport des K_{0,5} (α₂) / K_{0,5} (α₁) étant supérieur à 10⁴, l'affinité des composés de l'invention vis à vis des récepteurs α₂ est complétée par une grande sélectivité.

### EXEMPLE 6 : Test d'antagonisme α₂ in vivo

Les propriétés antagonistes α₂ des composés de l'invention ont été déterminées selon une technique décrite par F. Colpaert et Coll. (DRUG Development Research 7,125-140, 1986) en mesurant les effets produits par ces composés sur la perte du réflexe de redressement et l'exophthalmie induites chez le rat par la xylazine, agent agoniste connu des récepteurs α₂-adrénergiques au niveau central.

Le rétablissement du réflexe de redressement mesure le potentiel antagoniste α₂ des composés, le retour de l'oeil à une position normale mesure le potentiel antagoniste α₁.

Lors de ce test, le composé de l'exemple 2 présente à une ED₅₀ (α₂) égale à 5 mg/kg et une ED₅₀ (α₁) supérieure à 10 mg/kg ce qui confirme ses propriétés d'antagoniste α₂ sélectif.

### COMPOSITION PHARMACEUTIQUE

### EXEMPLE 7 : Comprimé : formule de préparation pour 1000 comprimés dosés à 2 mg

| | |
|---|---|
| 4-(2-cyclopentyl-3,3,3-trifluoropropan-1-yl)imidazole | 2 g |
| Hydroxypropyl cellulose | 2 g |
| Amidon de blé | 10 g |
| Lactose | 100 g |
| Stéarate de magnésium | 3 g |
| Talc | 3 g |

## Revendications (Revendications pour l'(les) Etat(s) contractant(s) suivant(s): AT, BE, CH, DE, DK, FR, GB, IT, LI, LU, NL, SE)

1. Composés de formule générale (I) : 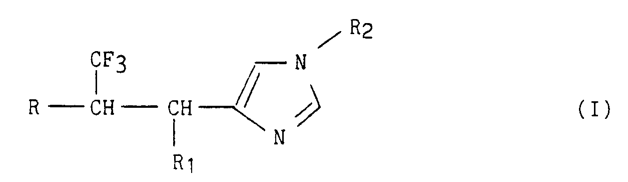 dans laquelle
R représente un groupement alkyle inférieur (C₁-C₆) linéaire ou ramifié, un groupement cycloalkyle (C₃-C₈), un groupement phényle (éventuellement substitué par un ou plusieurs atomes d'halogène, un ou plusieurs groupements alkyles (C₁-C₆) linéaires ou ramifiés eux-mêmes éventuellement substitués par un ou plusieurs atomes d'halogène, un ou plusieurs groupements alkoxy (C₁-C₆) linéaires ou ramifiés), un groupement pyridyle, un groupement naphtyl,
R₁ et R₂ identiques ou différents représentent un atome d'hydrogène, un groupement alkyle (C₁-C₆) linéaire ou ramifié, ou groupement acyle (C₁-C₆) linéaire ou ramifié,
leurs énantiomères, diastéréoisomères et épimères ainsi que leurs sels d'addition à un acide pharmaceutiquement acceptable.

2. Composés selon la revendication 1 tels que R représente un groupement cycloalkyle (C₃-C₈), leurs énantiomères, diastéréoisomères et épimères ainsi que leurs sels d'addition à un acide pharmaceutiquement acceptable.

3. Composés selon la revendication 1 tels que R₁ et R₂ représentent simultanément un atome d'hydrogène, leurs énantiomères, diastéréoisomères et épimères ainsi que leurs sels d'addition à un acide pharmaceutiquement acceptable.

4. Composé selon l'une quelconque des revendications 1, 2 ou 3 qui est le 4-(2-cyclopentyl-3,3,3-trifluoropropan-1-yl) imidazole, ses énantiomères ainsi que ses sels d'addition à un acide pharmaceutiquement acceptable.

5. Composé selon l'une quelconque des revendications 1 ou 3 qui est le 4-(2-phényl-3,3,3-trifluoropropan-1-yl) imidazole, ses énantiomères ainsi que ses sels d'addition à un acide pharmaceutiquement acceptable.

6. Procédé de préparation des composés de formule (I) selon la revendication 1 caractérisé en ce que l'on soumet un composé organomagnésien de formule (II)
R - MgX (II)
dans laquelle
R a la même signification que précédemment et X représente un atome d'halogène
à l'action de l'acide trifluoroacétique ou de son sel de sodium,
pour conduire à une cétone de formule (III) 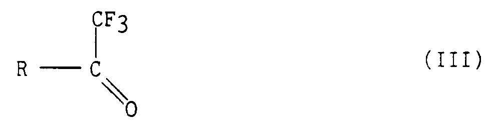 dans laquelle R a la même signification que dans la formule (I),
que l'on fait réagir avec les phosphonoacétates de formule (IV), en présence d'une base, 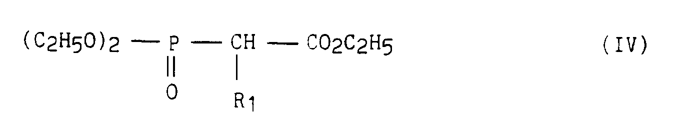 dans laquelle R₁ a la même signification que dans la formule (I),
pour conduire aux esters insaturés de formule (V) 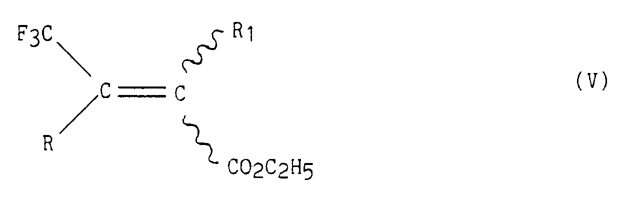 dans laquelle R et R₁ ont la même signification que dans la formule (I),
que l'on soumet à une hydrogénation catalytique en utilisant, par exemple, comme catalyseur du charbon palladié ou de l'oxyde de platine,
pour conduire aux esters de formule (VI) 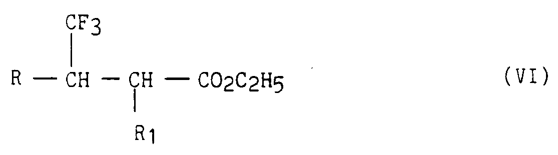 dans laquelle R et R₁ ont la même signification que dans la formule (I),
que l'on saponifie en présence de potasse ou de soude aqueuse,
pour conduire aux acides correspondants de formule (VII) 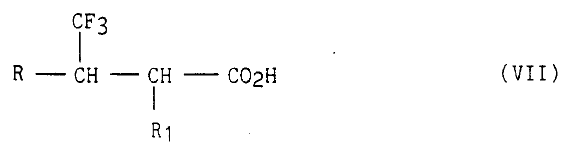 dans laquelle R et R₁ ont la même signification que dans la formule (I),
que l'on soumet à l'action du chlorure de thionyle,
pour conduire aux chlorures d'acide correspondants de formule (VIII) 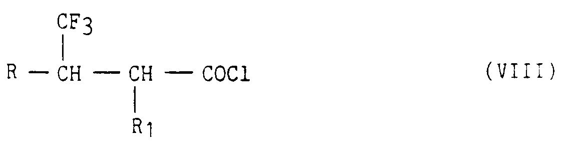 dans laquelle R et R₁ ont la même signification que dans la formule (I),
que l'on traite ensuite avec un excès de diazométhane,
pour conduire aux diazocétones de formule (IX) 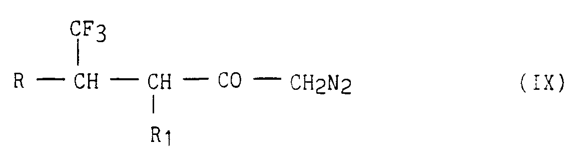 dans laquelle R et R₁ ont la même signification que dans la formule (I),
que l'on soumet à l'action de l'acide chlorhydrique gazeux,
pour conduire aux chlorocétones correspondantes de formule (X) 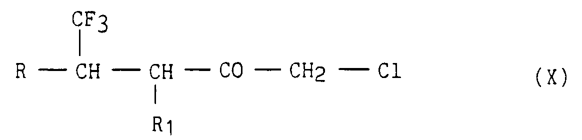 dans laquelle R et R₁ ont la même signification que dans la formule (I) que l'on fait réagir avec l'acétate de formamidine dans un solvant à haut point d'ébullition comme par exemple le diéthylène glycol,
pour conduire aux dérivés de formule (I),
que l'on purifie par des techniques classiques de purification, dont on sépare éventuellement les isomères selon les techniques classiques de séparation et que l'on transforme, si on le désire, en leurs sels d'addition à un acide pharmaceutiquement acceptable.

7. Composés de formule (VIII) selon la revendication 6, utiles pour la préparation des composés de formule (I) selon la revendication 1.

8. Composés de formule (IX) selon la revendication 6, utiles pour la préparation des composés de formule (I) selon la revendication 1.

9. Composés de formule (X) selon la revendication 6, utiles pour la préparation des composés de formule (I) selon la revendication 1.

10. Compositions pharmaceutiques contenant comme principe actif au moins un composé selon l'une quelconque des revendications 1 à 5, seul ou en combinaison avec un ou plusieurs excipients ou véhicules inertes non toxiques, pharmaceutiquement acceptables.

11. Compositions pharmaceutiques selon la revendication 10 contenant au moins un principe actif selon l'une quelconque des revendications 1 à 5 utiles, en tant qu'antagonistes α₂, dans le traitement de la dépression, de l'hypertension, de la maladie de Parkinson, des états convulsivants, de l'asthme, de l'aggrégation plaquettaire, du diabète et de l'obésité.

## Revendications (Revendications pour l'(les) Etat(s) contractant(s) suivant(s): ES)

1. Procédé de préparation des composés de formule générale (I) : 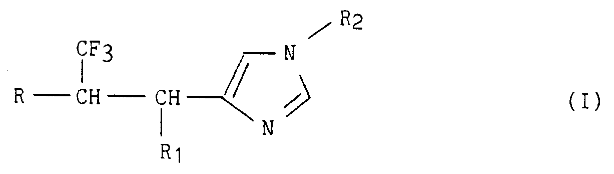 dans laquelle
R représente un groupement alkyle inférieur (C₁-C₆) linéaire ou ramifié, un groupement cycloalkyle (C₃-C₈), un groupement phényle (éventuellement substitué par un ou plusieurs atomes d'halogène, un ou plusieurs groupements alkyles (C₁-C₆) linéaires ou ramifiés eux-mêmes éventuellement substitués par un ou plusieurs atomes d'halogène, un ou plusieurs groupements alkoxy (C₁-C₆) linéaires ou ramifiés), un groupement pyridyle, un groupement naphtyl,
R₁ et R₂ identiques ou différents représentent un atome d'hydrogène, un groupement alkyle (C₁-C₆) linéaire ou ramifié, ou groupement acyle (C₁-C₆) linéaire ou ramifié,
de leurs énantiomères, diastéréoisomères et épimères ainsi que de leurs sels d'addition à un acide pharmaceutiquement acceptable,
caractérisé en ce que :
on soumet un composé organomagnésien de formule (II)
R - MgX (II)
dans laquelle
R a la même signification que précédemment et X représente un atome d'halogène
à l'action de l'acide trifluoroacétique ou de son sel de sodium,
pour conduire à une cétone de formule (III) 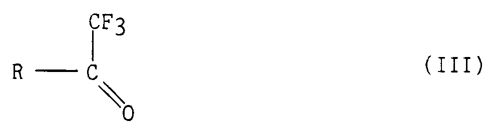 dans laquelle R a la même signification que dans la formule (I),
que l'on fait réagir avec les phosphonoacétates de formule (IV), en présence d'une base, 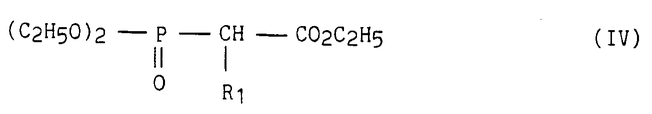 dans laquelle R₁ a la même signification que dans la formule (I),
pour conduire aux esters insaturés de formule (V) 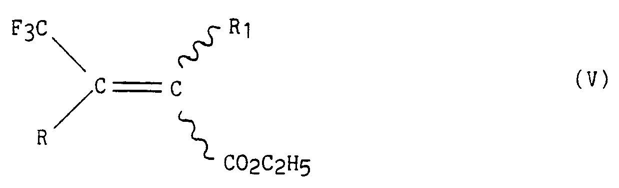 dans laquelle R et R₁ ont la même signification que dans la formule (I),
que l'on soumet à une hydrogénation catalytique en utilisant, par exemple, comme catalyseur du charbon palladié ou de l'oxyde de platine,
pour conduire aux esters de formule (VI) 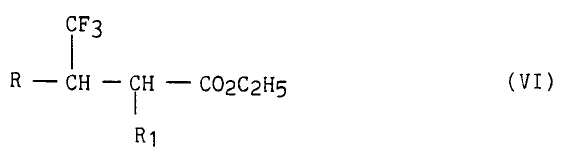 dans laquelle R et R₁ ont la même signification que dans la formule (I),
que l'on saponifie en présence de potasse ou de soude aqueuse,
pour conduire aux acides correspondants de formule (VII) 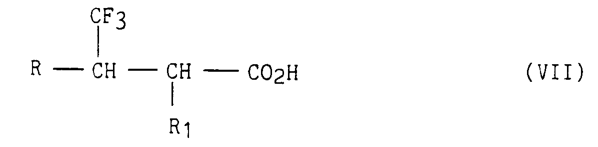 dans laquelle R et R₁ ont la même signification que dans la formule (I),
que l'on soumet à l'action du chlorure de thionyle,
pour conduire aux chlorures d'acide correspondants de formule (VIII) 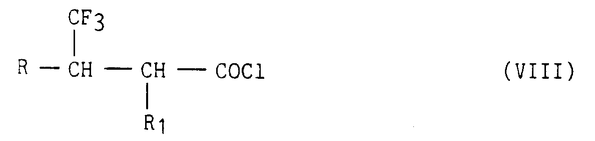 dans laquelle R et R₁ ont la même signification que dans la formule (I),
que l'on traite ensuite avec un excès de diazométhane,
pour conduire aux diazocétones de formule (IX) 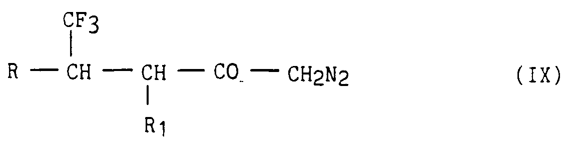 dans laquelle R et R₁ ont la même signification que dans la formule (I),
que l'on soumet à l'action de l'acide chlorhydrique gazeux,
pour conduire aux chlorocétones correspondantes de formule (X) 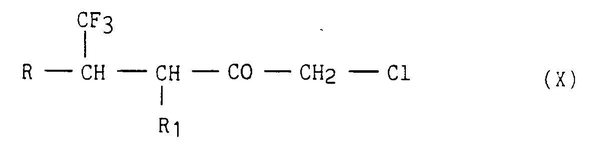 dans laquelle R et R₁ ont la même signification que dans la formule (I) que l'on fait réagir avec l'acétate de formamidine dans un solvant à haut point d'ébullition comme par exemple le diéthylène glycol,
pour conduire aux dérivés de formule (I),
que l'on purifie par des techniques classiques de purification, dont on sépare éventuellement les isomères selon les techniques classiques de séparation et que l'on transforme, si on le désire, en leurs sels d'addition à un acide pharmaceutiquement acceptable.

2. Procédé de préparation selon la revendication 1 des composés de formule (I) tels que R représente un groupement cycloalkyle (C₃-C₈), de leurs énantiomères, diastéréoisomères et épimères ainsi que de leurs sels d'addition à un acide pharmaceutiquement acceptable.

3. Procédé de préparation selon la revendication 1 des composés de formule (I) tels que R₁ et R₂ représentent simultanément un atome d'hydrogène, de leurs énantiomères, diastéréoisomères et épimères ainsi que de leurs sels d'addition à un acide pharmaceutiquement acceptable.

4. Procédé de préparation selon l'une quelconque des revendications 1, 2 ou 3 du 4-(2-cyclopentyl-3,3,3-trifluoropropan-1-yl) imidazole, de ses énantiomères ainsi que de ses sels d'addition à un acide pharmaceutiquement acceptable.

5. Procédé de préparation selon l'une quelconque des revendications 1 ou 3 du 4-(2-phényl-3,3,3-trifluoropropan-1-yl) imidazole, de ses énantiomères ainsi que de ses sels d'addition à un acide pharmaceutiquement acceptable.

6. Procédé de préparation selon la revendication 1 des composés de formule (VIII), utiles pour la préparation des composés de formule (I).

7. Procédé de préparation selon la revendication 1 des composés de formule (IX), utile dans le procédé de préparation des composés de formule (I).

8. Procédé de préparation selon la revendication 1 des composés de formule (X), utile dans le procédé de préparation des composés de formule (I).

## Revendications (Revendications pour l'(les) Etat(s) contractant(s) suivant(s): GR)

1. Procédé de préparation des composés de formule générale (I) : 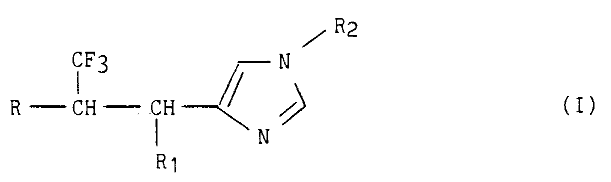 dans laquelle
R représente un groupement alkyle inférieur (C₁-C₆) linéaire ou ramifié, un groupement cycloalkyle (C₃-C₈), un groupement phényle (éventuellement substitué par un ou plusieurs atomes d'halogène, un ou plusieurs groupements alkyles (C₁-C₆) linéaires ou ramifiés eux-mêmes éventuellement substitués par un ou plusieurs atomes d'halogène, un ou plusieurs groupements alkoxy (C₁-C₆) linéaires ou ramifiés), un groupement pyridyle, un groupement naphtyl,
R₁ et R₂ identiques ou différents représentent un atome d'hydrogène, un groupement alkyle (C₁-C₆) linéaire ou ramifié, ou groupement acyle (C₁-C₆) linéaire ou ramifié,
de leurs énantiomères, diastéréoisomères et épimères ainsi que de leurs sels d'addition à un acide pharmaceutiquement acceptable,
caractérisé en ce que :
on soumet un composé organomagnésien de formule (II)
R - MgX (II)
dans laquelle
R a la même signification que précédemment et X représente un atome d'halogène
à l'action de l'acide trifluoroacétique ou de son sel de sodium,
pour conduire à une cétone de formule (III) 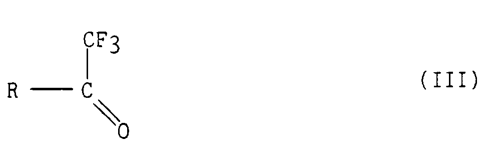 dans laquelle R a la même signification que dans la formule (I),
que l'on fait réagir avec les phosphonoacétates de formule (IV), en présence d'une base, 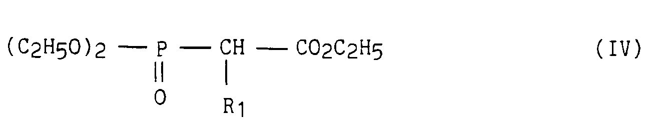 dans laquelle R₁ a la même signification que dans la formule (I),
pour conduire aux esters insaturés de formule (V) 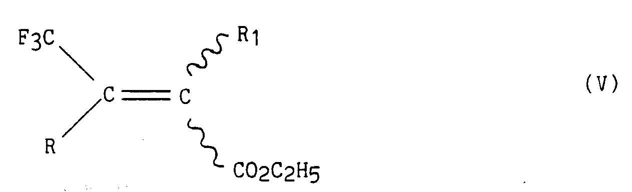 dans laquelle R et R₁ ont la même signification que dans la formule (I),
que l'on soumet à une hydrogénation catalytique en utilisant, par exemple, comme catalyseur du charbon palladié ou de l'oxyde de platine,
pour conduire aux esters de formule (VI) 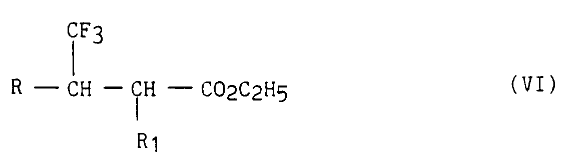 dans laquelle R et R₁ ont la même signification que dans la formule (I),
que l'on saponifie en présence de potasse ou de soude aqueuse,
pour conduire aux acides correspondants de formule (VII) 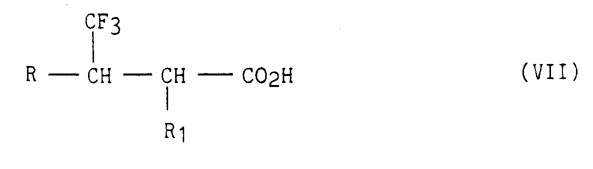 dans laquelle R et R₁ ont la même signification que dans la formule (I),
que l'on soumet à l'action du chlorure de thionyle,
pour conduire aux chlorures d'acide correspondants de formule (VIII) 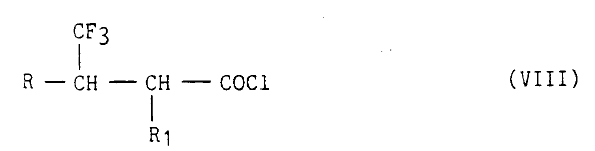 dans laquelle R et R₁ ont la même signification que dans la formule (I),
que l'on traite ensuite avec un excès de diazométhane,
pour conduire aux diazocétones de formule (IX) 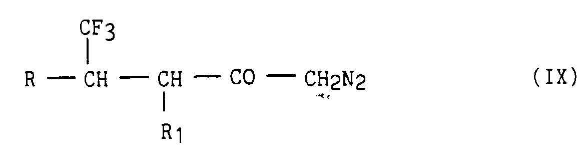 dans laquelle R et R₁ ont la même signification que dans la formule (I),
que l'on soumet à l'action de l'acide chlorhydrique gazeux,
pour conduire aux chlorocétones correspondantes de formule (X) 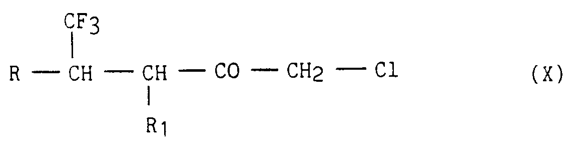 dans laquelle R et R₁ ont la même signification que dans la formule (I) que l'on fait réagir avec l'acétate de formamidine dans un solvant à haut point d'ébullition comme par exemple le diéthylène glycol,
pour conduire aux dérivés de formule (I),
que l'on purifie par des techniques classiques de purification, dont on sépare éventuellement les isomères selon les techniques classiques de séparation et que l'on transforme, si on le désire, en leurs sels d'addition à un acide pharmaceutiquement acceptable.

2. Procédé de préparation selon la revendication 1 des composés de formule (I) tels que R représente un groupement cycloalkyle (C₃-C₈), de leurs énantiomères, diastéréoisomères et épimères ainsi que de leurs sels d'addition à un acide pharmaceutiquement acceptable.

3. Procédé de préparation selon la revendication 1 des composés de formule (I) tels que R₁ et R₂ représentent simultanément un atome d'hydrogène, de leurs énantiomères, diastéréoisomères et épimères ainsi que de leurs sels d'addition à un acide pharmaceutiquement acceptable.

4. Procédé de préparation selon l'une quelconque des revendications 1, 2 ou 3 du 4-(2-cyclopentyl-3,3,3-trifluoropropan-1-yl) imidazole, de ses énantiomères ainsi que de ses sels d'addition à un acide pharmaceutiquement acceptable.

5. Procédé de préparation selon l'une quelconque des revendications 1 ou 3 du 4-(2-phényl-3,3,3-trifluoropropan-1-yl) imidazole, de ses énantiomères ainsi que de ses sels d'addition à un acide pharmaceutiquement acceptable.

6. Procédé de préparation selon la revendication 1 des composés de formule (VIII), utiles pour la préparation des composés de formule (I).

7. Procédé de préparation selon la revendication 1 des composés de formule (IX), utile dans le procédé de préparation des composés de formule (I).

8. Procédé de préparation selon la revendication 1 des composés de formule (X), utile dans le procédé de préparation des composés de formule (I).

## Claims (Claims for the following Contracting State(s): AT, BE, CH, DE, DK, FR, GB, IT, LI, LU, NL, SE)

1. Compounds of the general formula (I): 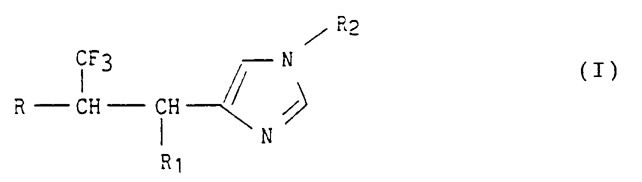 in which
R represents a linear or branched (C₁-C₆)-lower alkyl group, a (C₃-C₈)-cycloalkyl group, a phenyl group (optionally substituted by one or more halogen atoms; one or more linear or branched (C₁-C₆)-alkyl groups which are themselves optionally substituted by one or more halogen atoms; or by one or more linear or branched (C₁-C₆)-alkoxy groups), a pyridyl group or a naphthyl group, and
R₁ and R₂, which may be the same or different, represent a hydrogen atom, a linear or branched (C₁-C₆)-alkyl group or a linear or branched (C₁-C₆)-acyl group,
their enantiomers, diastereoisomers and epimers, and their addition salts with a pharmaceutically acceptable acid.

2. Compounds according to claim 1 in which R represents a (C₃-C₈)-cycloalkyl group, their enantiomers, diastereoisomers and epimers, and their addition salts with a pharmaceutically acceptable acid.

3. Compounds according to claim 1 in which R₁ and R₂ simultaneously represent a hydrogen atom, their enantiomers, diastereoisomers and epimers, and their addition salts with a pharmaceutically acceptable acid.

4. Compound according to any one of claims 1, 2 and 3 which is 4-(2-cyclopentyl-3,3,3-trifluoropropan-1-yl)imidazole, its enantiomers and its addition salts with a pharmaceutically acceptable acid.

5. Compound according to either claim 1 or claim 3 which is 4-(2-phenyl-3,3,3-trifluoropropan-1-yl)imidazole, its enantiomers and its addition salts with a pharmaceutically acceptable acid.

6. Process for the preparation of the compounds of formula (I) according to claim 1, characterised in that an organomagnesium compound of formula (II)
R-MgX (II),
in which R has the same meaning as above and X represents a halogen atom,
is subjected to the action of trifluoroacetic acid or its sodium salt to give a ketone of formula (III) 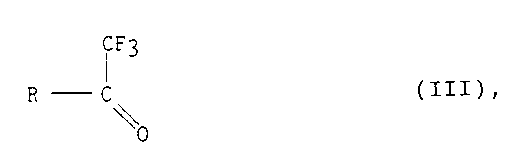 in which R has the same meaning as in formula (I),
which is reacted, in the presence of a base, with the phosphonoacetates of formula (IV) 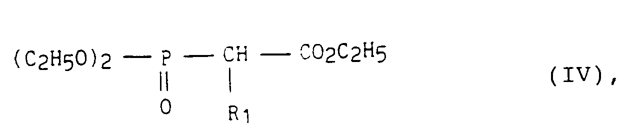 in which R₁ has the same meaning as in formula (I),
to give the unsaturated esters of formula (V) 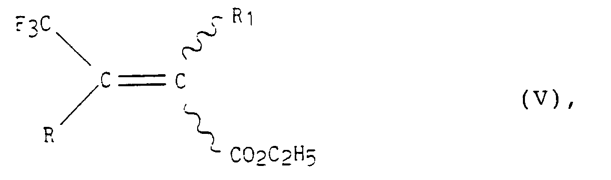 in which R and R₁ have the same meaning as in formula (I),
which are subjected to catalytic hydrogenation using, for example, palladium-on-carbon or platinum oxide as catalyst, to give the esters of formula (VI) 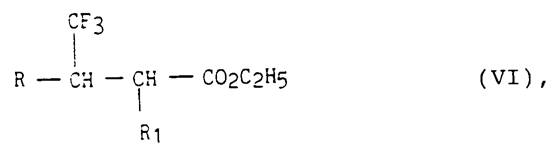 in which R and R₁ have the same meaning as in formula (I),
which are hydrolysed in the presence of aqueous potassium hydroxide or sodium hydroxide to give the corresponding acids of formula (VII) 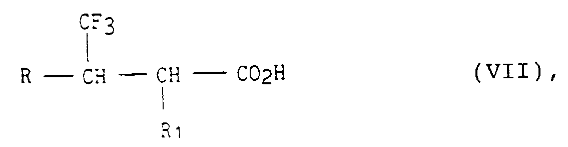 in which R and R₁ have the same meaning as in formula (I),
which are subjected to the action of thionyl chloride to give the corresponding acid chlorides of formula (VIII) 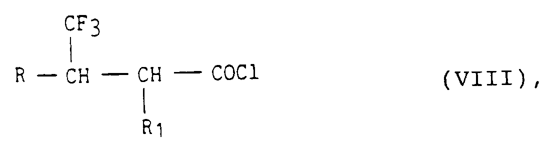 in which R and R₁ have the same meaning as in formula (I),
which are then treated with an excess of diazomethane to give the diazoketones of formula (IX) 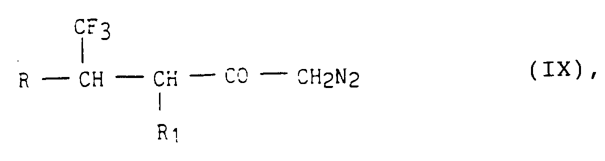 in which R and R₁ have the same meaning as in formula (I),
which are subjected to the action of gaseous hydrochloric acid to give the corresponding chloroketones of formula (X) 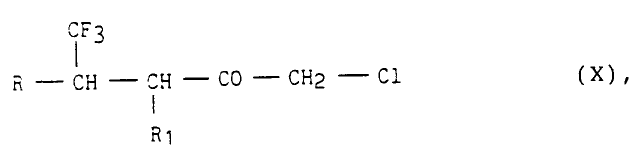 in which R and R₁ have the same meaning as in formula (I),
which are reacted with formamidine acetate in a solvent having a high boiling point, such as, for example,
diethylene glycol, to give the compounds of formula (I), which are purified by conventional purification methods, the isomers of which are optionally separated by conventional separation methods, and which are converted, if desired, into their addition salts with a pharmaceutically acceptable acid.

7. Compounds of formula (VIII) according to claim 6 which can be used for the preparation of the compounds of formula (I) according to claim 1.

8. Compounds of formula (IX) according to claim 6 which can be used for the preparation of the compounds of formula (I) according to claim 1.

9. Compounds of formula (X) according to claim 6 which can be used for the preparation of the compounds of formula (I) according to claim 1.

10. Pharmaceutical compositions containing as active ingredient at least one compound according to any one of claims 1 to 5, on its own or in combination with one or more inert, non-toxic, pharmaceutically acceptable excipients or carriers.

11. Pharmaceutical compositions according to claim 10 containing at least one active ingredient according to any one of claims 1 to 5, for use, as α₂ antagonists, in the treatment of depression, hypertension, Parkinson's disease, convulsive conditions, asthma, platelet aggregation, diabetes and obesity.

## Claims (Claims for the following Contracting State(s): ES)

1. Process for the preparation of compounds of the general formula (I): 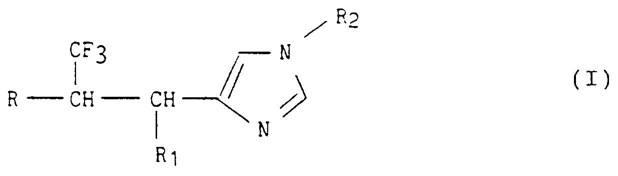 in which
R represents a linear or branched (C₁-C₆)-lower alkyl group, a (C₃-C₈)-cycloalkyl group, a phenyl group (optionally substituted by one or more halogen atoms; one or more linear or branched (C₁-C₆)-alkyl groups which are themselves optionally substituted by one or more halogen atoms; or by one or more linear or branched (C₁-C₆)-alkoxy groups), a pyridyl group or a naphthyl group, and
R₁ and R₂, which may be the same or different, represent a hydrogen atom, a linear or branched (C₁-C₆)-alkyl group or a linear or branched (C₁-C₆)-acyl group,
their enantiomers, diastereoisomers and epimers, and their addition salts with a pharmaceutically acceptable acid,
characterised in that:
an organomagnesium compound of formula (II)
R-MgX (II),
in which R has the same meaning as above and X represents a halogen atom,
is subjected to the action of trifluoroacetic acid or its sodium salt to give a ketone of formula (III) 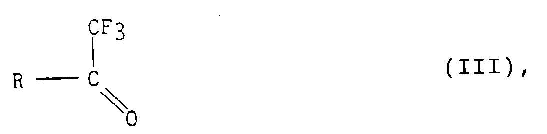 in which R has the same meaning as in formula (I),
which is reacted, in the presence of a base, with the phosphonoacetates of formula (IV) 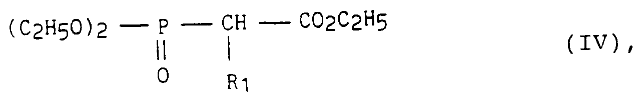 in which R₁ has the same meaning as in formula (I),
to give the unsaturated esters of formula (V) 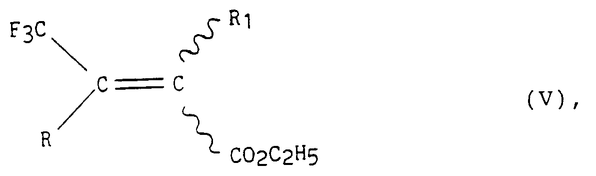 in which R and R₁ have the same meaning as in formula (I),
which are subjected to catalytic hydrogenation using, for example, palladium-on-carbon or platinum oxide as catalyst, to give the esters of formula (VI) 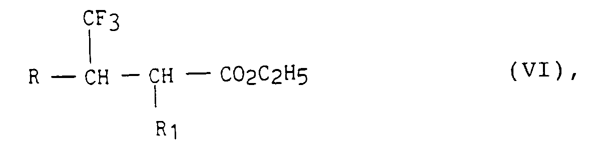 in which R and R₁ have the same meaning as in formula (I),
which are hydrolysed in the presence of aqueous potassium hydroxide or sodium hydroxide to give the corresponding acids of formula (VII) 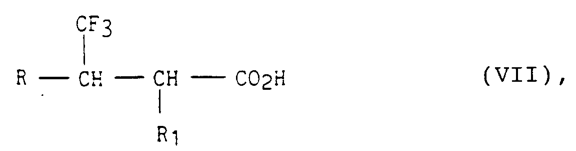 in which R and R₁ have the same meaning as in formula (I),
which are subjected to the action of thionyl chloride to give the corresponding acid chlorides of formula (VIII) 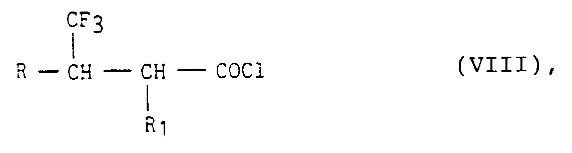 in which R and R₁ have the same meaning as in formula (I),
which are then treated with an excess of diazomethane to give the diazoketones of formula (IX) 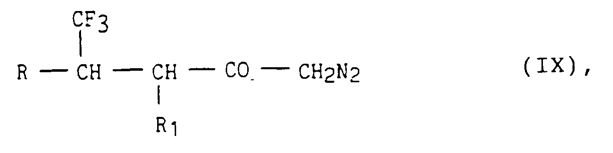 in which R and R₁ have the same meaning as in formula (I),
which are subjected to the action of gaseous hydrochloric acid to give the corresponding chloroketones of formula (X) 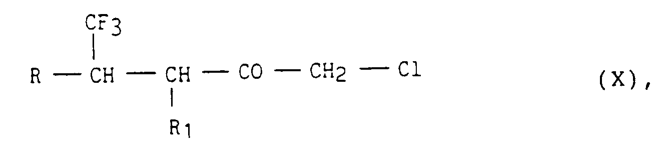 in which R and R₁ have the same meaning as in formula (I),
which are reacted with formamidine acetate in a solvent having a high boiling point, such as, for example,
diethylene glycol, to give the compounds of formula (I), which are purified by conventional purification methods, the isomers of which are optionally separated by conventional separation methods, and which are converted, if desired, into their addition salts with a pharmaceutically acceptable acid.

2. Process according to claim 1 for the preparation of compounds of formula (I) in which R represents a (C₃-C₈)-cycloalkyl group, their enantiomers, diastereoisomers and epimers, and their addition salts with a pharmaceutically acceptable acid.

3. Process according to claim 1 for the preparation of compounds of formula (I) in which R₁ and R₂ simultaneously represent a hydrogen atom, their enantiomers, diastereoisomers and epimers, and their addition salts with a pharmaceutically acceptable acid.

4. Process according to any one of claims 1, 2 and 3 for the preparation of 4-(2-cyclopentyl-3,3,3-trifluoropropan-1-yl)imidazole, its enantiomers and its addition salts with a pharmaceutically acceptable acid.

5. Process according to either claim 1 or claim 3 for the preparation of 4-(2-phenyl-3,3,3-trifluoropropan-1-yl)imidazole, its enantiomers and its addition salts with a pharmaceutically acceptable acid.

6. Process according to claim 1 for the preparation of compounds of formula (VIII) which can be used for the preparation of the compounds of formula (I).

7. Process according to claim 1 for the preparation of compounds of formula (IX) which can be used in the process for the preparation of the compounds of formula (I).

8. Process according to claim 1 for the preparation of compounds of formula (X) which can be used in the process for the preparation of the compounds of formula (I).

## Claims (Claims for the following Contracting State(s): GR)

1. Process for the preparation of compounds of the general formula (I): 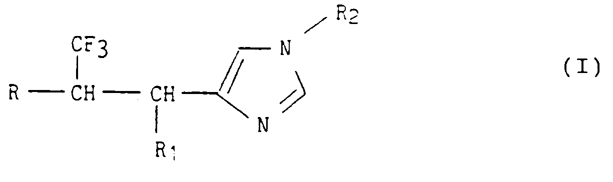 in which
R represents a linear or branched (C₁-C₆)-lower alkyl group, a (C₃-C₈)-cycloalkyl group, a phenyl group (optionally substituted by one or more halogen atoms; one or more linear or branched (C₁-C₆)-alkyl groups which are themselves optionally substituted by one or more halogen atoms; or by one or more linear or branched (C₁-C₆)-alkoxy groups), a pyridyl group or a naphthyl group, and
R₁ and R₂, which may be the same or different, represent a hydrogen atom, a linear or branched (C₁-C₆)-alkyl group or a linear or branched (C₁-C₆)-acyl group,
their enantiomers, diastereoisomers and epimers, and their addition salts with a pharmaceutically acceptable acid,
characterised in that:
an organomagnesium compound of formula (II)
R-MgX (II),
in which R has the same meaning as above and X represents a halogen atom,
is subjected to the action of trifluoroacetic acid or its sodium salt to give a ketone of formula (III) 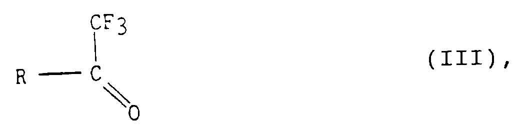 in which R has the same meaning as in formula (I),
which is reacted, in the presence of a base, with the phosphonoacetates of formula (IV) 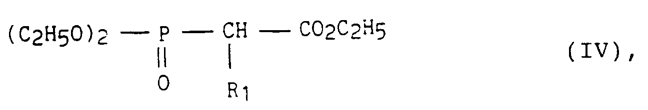 in which R₁ has the same meaning as in formula (I),
to give the unsaturated esters of formula (V) 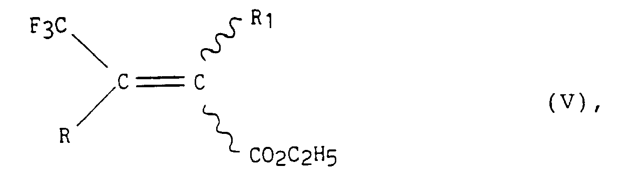 in which R and R₁ have the same meaning as in formula (I),
which are subjected to catalytic hydrogenation using, for example, palladium-on-carbon or platinum oxide as catalyst, to give the esters of formula (VI) 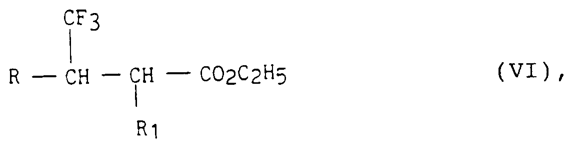 in which R and R₁ have the same meaning as in formula (I),
which are hydrolysed in the presence of aqueous potassium hydroxide or sodium hydroxide to give the corresponding acids of formula (VII) 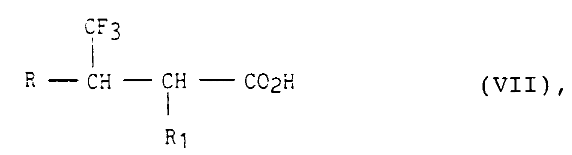 in which R and R₁ have the same meaning as in formula (I),
which are subjected to the action of thionyl chloride to give the corresponding acid chlorides of formula (VIII) 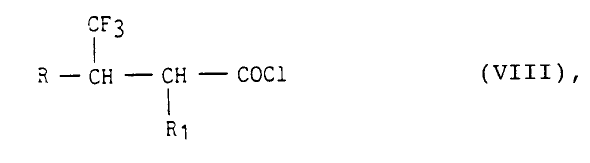 in which R and R₁ have the same meaning as in formula (I),
which are then treated with an excess of diazomethane to give the diazoketones of formula (IX) 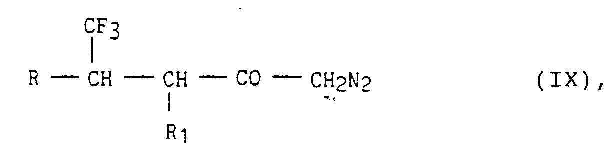 in which R and R₁ have the same meaning as in formula (I),
which are subjected to the action of gaseous hydrochloric acid to give the corresponding chloroketones of formula (X) 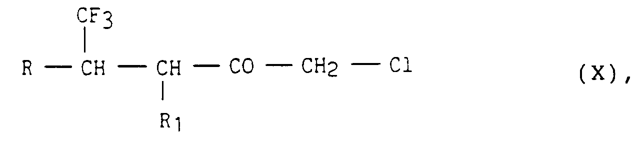 in which R and R₁ have the same meaning as in formula (I),
which are reacted with formamidine acetate in a solvent having a high boiling point, such as, for example, diethylene glycol, to give the compounds of formula (I), which are purified by conventional purification methods, the isomers of which are optionally separated by conventional separation methods, and which are converted, if desired, into their addition salts with a pharmaceutically acceptable acid.

2. Process according to claim 1 for the preparation of compounds of formula (I) in which R represents a (C₃-C₈)-cycloalkyl group, their enantiomers, diastereoisomers and epimers, and their addition salts with a pharmaceutically acceptable acid.

3. Process according to claim 1 for the preparation of compounds of formula (I) in which R₁ and R₂ simultaneously represent a hydrogen atom, their enantiomers, diastereoisomers and epimers, and their addition salts with a pharmaceutically acceptable acid.

4. Process according to any one of claims 1, 2 and 3 for the preparation of 4-(2-cyclopentyl-3,3,3-trifluoropropan-1-yl)imidazole, its enantiomers and its addition salts with a pharmaceutically acceptable acid.

5. Process according to either claim 1 or claim 3 for the preparation of 4-(2-phenyl-3,3,3-trifluoropropan-1-yl)imidazole, its enantiomers and its addition salts with a pharmaceutically acceptable acid.

6. Process according to claim 1 for the preparation of compounds of formula (VIII) which can be used for the preparation of the compounds of formula (I).

7. Process according to claim 1 for the preparation of compounds of formula (IX) which can be used in the process for the preparation of the compounds of formula (I).

8. Process according to claim 1 for the preparation of compounds of formula (X) which can be used in the process for the preparation of the compounds of formula (I).

## Patentansprüche (Patentansprüche für folgende(n) Vertragsstaat(en): AT, BE, CH, DE, DK, FR, GB, IT, LI, LU, NL, SE)

1. Verbindungen der allgemeinen Formel I: 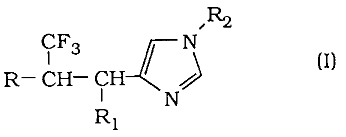 in der
R eine geradkettige oder verzweigte niedrigmolekulare (C₁-C₆)-Alkylgruppe, eine (C₃-C₈)-Cycloalkylgruppe, eine Phenylgruppe (die gegebenenfalls durch eines oder mehrere Halogenatome oder eine oder mehrere geradkettige oder verzweigte (C₁-C₆)-Alkylgruppen substituiert ist, welche ihrerseits gegebenenfalls durch eines oder mehrere Halogenatome oder eine oder mehrere geradkettige oder verzweigte (C₁-C₆)-Alkoxygruppen substituiert sind), eine Pyridylgruppe oder eine Naphthylgruppe und
R₁ und R₂, die gleichartig oder verschieden sein können, Wasserstoffatome, geradkettige oder verzweigte (C₁-C₆)-Alkylgruppen oder geradkettige oder verzweigte (C₁-C₆)-Acylgruppen bedeuten,
deren Enantiomere, Diastereoisomere und Epimere sowie deren Additionssalze mit einer pharmazeutisch annehmbaren Säure.

2. Verbindungen nach Anspruch 1, worin R eine (C₃-C₈)-Cycloalkylgruppe darstellt, deren Enantiomere, Diastereoisomere und Epimere sowie ihre Additionssalze mit einer pharmazeutisch annehmbaren Säure.

3. Verbindungen nach Anspruch 1, worin R₁ und R₂ gleichzeitig Wasserstoffatome bedeuten, deren Enantiomere, Diastereoisomere und Epimere sowie deren Additionssalze mit einer pharmazeutisch annehmbaren Säure.

4. Verbindung nach einem der Ansprüche 1, 2 oder 3, nämlich 4-(2-Cyclopentyl-3,3,3-trifluorpropan-1-yl)-imidazol, dessen Enantiomere sowie dessen Additionssalze mit einer pharmazeutisch annehmbaren Säure.

5. Verbindung nach einem der Ansprüche 1 oder 3, nämlich 4-(2-Phenyl-3,3,3-trifluorpropan-1-yl)-imidazol, dessen Enantiomere sowie dessen Additionssalze mit einer pharmazeutisch annehmbaren Säure.

6. Verfahren zur Herstellung der Verbindungen der Formel (I) nach Anspruch 1, **dadurch gekennzeichnet**, daß man eine magnesiumorganische Verbindung der Formel (II)
R - MgX (II)
in der
R die oben angegebenen Bedeutungen besitzt und X ein Halogenatom darstellt, der Einwirkung von Trifluoressigsäure oder seines Natriumsalzes unterwirft, so daß man ein Keton der Formel (III) 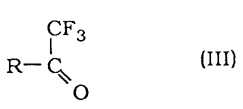 erhält, in der R die bezüglich der Formel (I) angegebenen Bedeutungen besitzt, welches man mit Phosphonoacetaten der Formel (IV) 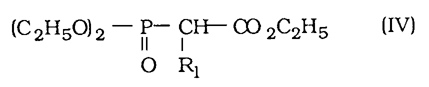 in der R₁ die bezüglich der Formel (I) angegebenen Bedeutungen besitzt, in Gegenwart einer Base umsetzt zur Bildung der ungesättigten Ester der Formel (V) 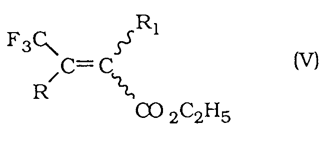 in der R und R₁ die bezüglich der Formel (I) angegebenen Bedeutungen besitzen, welche man einer katalytischen Hydrierung unter Verwendung von beispielsweise Palladium-auf-Kohlenstoff oder Platinoxid als Katalysator unterwirft zur Bildung der Ester der Formel (VI) 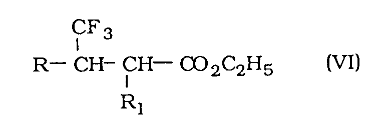 in der Rund R₁ die bezüglich der Formel (I) angegebenen Bedeutungen besitzen, welche man in Gegenwart von wäßrigem Kaliumhydroxid oder Natriumhydroxid verseift zur Bildung der entsprechenden Säuren der Formel (VII) 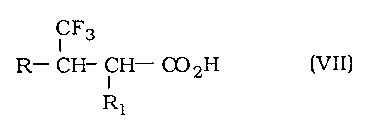 in der Rund R₁ die bezüglich der Formel (I) angegebenen Bedeutungen besitzen, welche man der Einwirkung von Thionylchlorid unterwirft zur Bildung der entsprechenden Säurechloride der Formel (VIII) 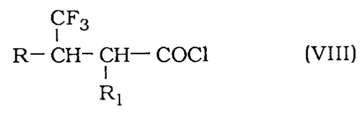 in der R und R₁ die bezüglich der Formel (I) angegebenen Bedeutungen besitzen, weiche man anschließend mit einem Überschuß von Diazomethan behandelt zur Bildung der Diazoketone der Formel (IX) 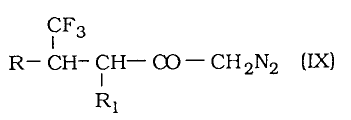 worin R und R₁ die bezüglich der Formel (I) angegebenen Bedeutungen besitzen, welche man der Einwirkung von gasförmiger Chlorwasserstoffsäure unterwirft zur Bildung der entsprechenden Chlorketone der Formel (X) 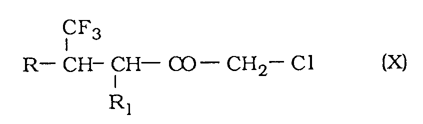 in der R und R₁ die bezüglich der Formel (I) angegebenen Bedeutungen besitzen, welche man mit Formamidinacetat in einem Lösungsmittel mit hohem Siedepunkt, wie beispielsweise Diethylenglykol, umsetzt zur Bildung der Derivate der Formel (I),
welche man durch Anwendung klassischer Reinigungsverfahren reinigt, gegebenenfalls unter Anwendung klassischer Trennungsmethode in die Isomeren auftrennt und die man gewünschtenfalls durch Zugabe einer pharmazeutisch annehmbaren Säure in ihre Salze überführt.

7. Für die Herstellung der Verbindungen der Formel (I) nach Anspruch 1 geeignete Verbindungen der Formel (VIII) gemäß Anspruch 6.

8. Für die Herstellung der Verbindungen der Formel (I) nach Anspruch 1 geeignete Verbindungen der Formel (IX) gemäß Anspruch 6.

9. Für die Herstellung der Verbindungen der Formel (I) nach Anspruch 1 geeignete Verbindungen der Formel (X) gemäß Anspruch 6.

10. Pharmazeutische Zubereitungen enthaltend als Wirkstoffmindestens eine Verbindung nach einem der Ansprüche 1 bis 5 allein oder in Kombination mit einem oder mehreren inerten, nichttoxischen, pharmazeutisch annehmbaren Trägermaterialien oder Bindemitteln.

11. Pharmazeutische Zubereitungen nach Anspruch 10 enthaltend mindestens einen Wirkstoff nach einem der Ansprüche 1 bis 5, geeignet als α₂-Antagonisten bei der Behandlung von Depressionen, der Hypertension, der Parkinsonschen Krankheit, von Krampfzuständen, des Asthmas, der Plättchenaggregation, des Diabetes und der Fettsucht.

## Patentansprüche (Patentansprüche für folgende(n) Vertragsstaat(en): ES)

1. Verfahren zur Herstellung der Verbindungen der allgemeinen Formel (I): 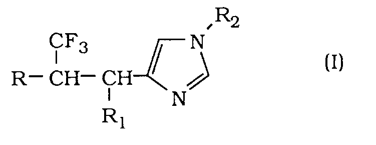 in der
R eine geradkettige oder verzweigte niedrigmolekulare (C₁-C₆)-Alkylgruppe, eine (C₃-C₈)-Cycloalkylgruppe, eine Phenylgruppe (die gegebenenfalls durch eines oder mehrere Halogenatome oder eine oder mehrere geradkettige oder verzweigte (C₁-C₆)-Alkylgruppen substituiert ist, welche ihrerseits gegebenenfalls durch eines oder mehrere Halogenatome oder eine oder mehrere geradkettige oder verzweigte (C₁-C₆)-Alkoxygruppen substituiert sind), eine Pyridylgruppe oder eine Naphthylgruppe und
R₁ und R₂, die gleichartig oder verschieden sein können, Wasserstoffatome, geradkettige oder verzweigte (C₁-C₆)-Alkylgruppen oder geradkettige oder verzweigte (C₁-C₆)-Acylgruppen bedeuten,
von deren Enantiomeren, Diastereoisomeren und Epimeren sowie von deren Additionssalzen mit einer pharmazeutisch annehmbaren Säure, **dadurch gekennzeichnet**, daß man eine magnesiumorganische Verbindung der Formel (II)
R - MgX (II)
in der
R die oben angegebenen Bedeutungen besitzt und X ein Halogenatom darstellt, der Einwirkung von Trifluoressigsäure oder seines Natriumsalzes unterwirft, so daß man ein Keton der Formel (III) 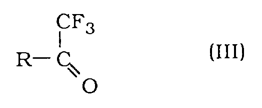 erhält, in der R die bezüglich der Formel (I) angegebenen Bedeutungen besitzt, welches man mit Phosphonoacetaten der Formel (IV) 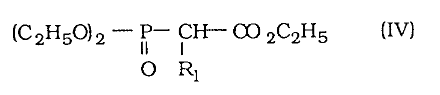 in der R₁ die bezüglich der Formel (I) angegebenen Bedeutungen besitzt,
in Gegenwart einer Base umsetzt zur Bildung der ungesättigten Ester der Formel (V) 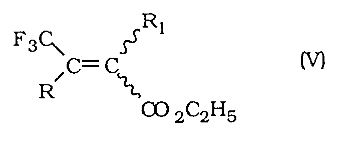 in der R und R₁ die bezüglich der Formel (I) angegebenen Bedeutungen besitzen, welche man einer katalytischen Hydrierung unter Verwendung von beispielsweise Palladium-auf-Kohlenstoff oder Platinoxid als Katalysator unterwirft zur Bildung der Ester der Formel (VI) 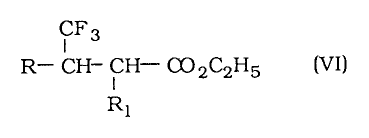 in der R und R₁ die bezüglich der Formel (I) angegebenen Bedeutungen besitzen, welche man in Gegenwart von wäßrigem Kaliumhydroxid oder Natriumhydroxid verseift zur Bildung der entsprechenden Säuren der Formel (VII) 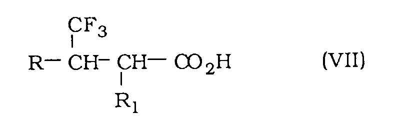 in der R und R₁ die bezüglich der Formel (I) angegebenen Bedeutungen besitzen, welche man der Einwirkung von Thionylchlorid unterwirft zur Bildung der entsprechenden Säurechloride der Formel (VIII) 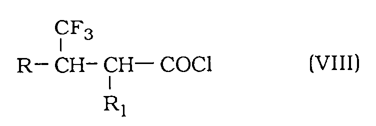 in der R und R₁ die bezüglich der Formel (I) angegebenen Bedeutungen besitzen, welche man anschließend mit einem Überschuß von Diazomethan behandelt zur Bildung der Diazoketone der Formel (IX) 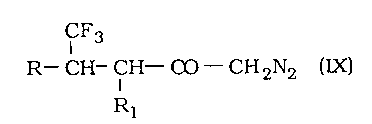 worin R und R₁ die bezüglich der Formel (I) angegebenen Bedeutungen besitzen, welche man der Einwirkung von gasförmiger Chlorwasserstoffsäure unterwirft zur Bildung der entsprechenden Chlorketone der Formel (X) 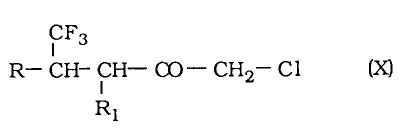 in der R und R₁ die bezüglich der Formel (I) angegebenen Bedeutungen besitzen, welche man mit Formamidinacetat in einem Lösungsmittel mit hohem Siedepunkt, wie beispielsweise Diethylenglykol, umsetzt zur Bildung der Derivate der Formel (I),
welche man durch Anwendung klassischer Reinigungsverfahren reinigt, gegebenenfalls unter Anwendung klassischer Trennungsmethode in die Isomeren auftrennt und die man gewünschtenfalls durch Zugabe einer pharmazeutisch annehmbaren Säure in ihre Salze überführt.

2. Verfahren nach Anspruch 1 zur Herstellung von Verbindungen der Formel (I), worin R eine (C₃-C₈)-Cycloalkylgruppe darstellt, von deren Enantiomeren, Diastereoisomeren und Epimeren sowie von deren Additionssalzen mit einer pharmazeutisch annehmbaren Säure.

3. Verfahren nach Anspruch 1 zur Herstellung von Verbindungen der Formel (I), worin R₁ und R₂ gleichzeitig Wasserstoffatome bedeuten, von deren Enantiomeren, Diastereoisomeren und Epimeren sowie von deren Additionssalzen mit einer pharmazeutisch annehmbaren Säure.

4. Verfahren nach einem der Ansprüche 1, 2 oder 3 zur Herstellung von 4-(2-Cyclopentyl-3,3,3-trifluorpropan-1-yl)-imidazol, von dessen Enantiomeren sowie von dessen Additionssalzen mit einer pharmazeutisch annehmbaren Säure.

5. Verfahren nach einem der Ansprüche 1 oder 3 zur Herstellung von 4-(2-Phenyl-3,3,3-trifluorpropan-1-yl)-imidazol und von dessen Enantiomeren sowie von dessen Additionssalzen mit einer pharmazeutisch annehmbaren Säure.

6. Verfahren nach Anspruch 1 zur Herstellung von Verbindungen der Formel (VIII) die für die Herstellung der Verbindungen der Formel (I) nützlich sind.

7. Verfahren nach Anspruch 1 zur Herstellung von Verbindungen der Formel (IX), die für die Herstellung der Verbindungen der Formel (I) nützlich sind.

8. Verfahren nach Anspruch 1 zur Herstellung von Verbindungen der Formel (X), die für die Herstellung der Verbindungen der Formel (I) nützlich sind.

## Patentansprüche (Patentansprüche für folgende(n) Vertragsstaat(en): GR)

1. Verfahren zur Herstellung der Verbindungen der allgemeinen Formel (I): 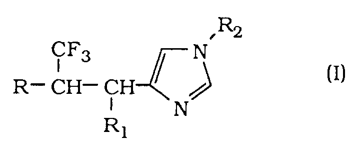 in der
R eine geradkettige oder verzweigte niedrigmolekulare (C₁-C₆)-Alkylgruppe, eine (C₃-C₈)-Cycloalkylgruppe, eine Phenylgruppe (die gegebenenfalls durch eines oder mehrere Halogenatome oder eine oder mehrere geradkettige oder verzweigte (C₁-C₆)-Alkylgruppen substituiert ist, welche ihrerseits gegebenenfalls durch eines oder mehrere Halogenatome oder eine oder mehrere geradkettige oder verzweigte (C₁-C₆)-Alkoxygruppen substituiert sind), eine Pyridylgruppe oder eine Naphthylgruppe und
R₁ und R₂, die gleichartig oder verschieden sein können, Wasserstoffatome, geradkettige oder verzweigte (C₁-C₆)-Alkylgruppen oder geradkettige oder verzweigte (C₁-C₆)-Acylgruppen bedeuten,
von deren Enantiomeren, Diastereoisomeren und Epimeren sowie von deren Additionssalzen mit einer pharmazeutisch annehmbaren Säure, **dadurch gekennzeichnet**, daß man eine magnesiumorganische Verbindung der Formel (II)
R - MgX (II)
in der
R die oben angegebenen Bedeutungen besitzt und X ein Halogenatom darstellt, der Einwirkung von Trifluoressigsäure oder seines Natriumsalzes unterwirft, so daß man ein Keton der Formel (III) 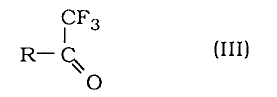 erhält, in der R die bezüglich der Formel (I) angegebenen Bedeutungen besitzt, welches man mit Phosphonoacetaten der Formel (IV) 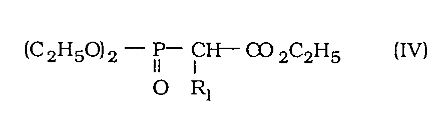 in der R₁ die bezüglich der Formel (I) angegebenen Bedeutungen besitzt, in Gegenwart einer Base umsetzt zur Bildung der ungesättigten Ester der Formel (V) 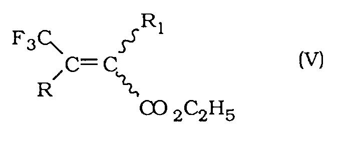 in der R und R₁ die bezüglich der Formel (I) angegebenen Bedeutungen besitzen, welche man einer katalytischen Hydrierung unter Verwendung von beispielsweise Palladium-auf-Kohlenstoff oder Platinoxid als Katalysator unterwirft zur Bildung der Ester der Formel (VI) 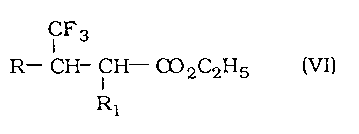 in der R und R₁ die bezüglich der Formel (I) angegebenen Bedeutungen besitzen, welche man in Gegenwart von wäßrigem Kaliumhydroxid oder Natriumhydroxid verseift zur Bildung der entsprechenden Säuren der Formel (VII) 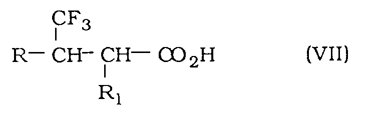 in der R und R₁ die bezüglich der Formel (I) angegebenen Bedeutungen besitzen, welche man der Einwirkung von Thionylchlorid unterwirft zur Bildung der entsprechenden Säurechloride der Formel (VIII) 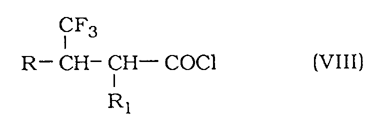 in der R und R₁ die bezüglich der Formel (I) angegebenen Bedeutungen besitzen, welche man anschließend mit einem Überschuß von Diazomethan behandelt zur Bildung der Diazoketone der Formel (IX) 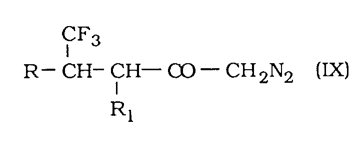 worin R und R₁ die bezüglich der Formel (I) angegebenen Bedeutungen besitzen, welche man der Einwirkung von gasförmiger Chlorwasserstoffsäure unterwirft zur Bildung der entsprechenden Chlorketone der Formel (X) 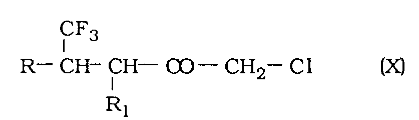 in der R und R₁ die bezüglich der Formel (I) angegebenen Bedeutungen besitzen, welche man mit Formamidinacetat in einem Lösungsmittel mit hohem Siedepunkt, wie beispielsweise Diethylenglykol, umsetzt zur Bildung der Derivate der Formel (I),
welche man durch Anwendung klassischer Reinigungsverfahren reinigt, gegebenenfalls unter Anwendung klassischer Trennungsmethode in die Isomeren auftrennt und die man gewünschtenfalls durch Zugabe einer pharmazeutisch annehmbaren Säure in Ihre Salze überführt.

2. Verfahren nach Anspruch 1 zur Herstellung von Verbindungen der Formel (I), worin R eine (C₃-C₈)-Cycloalkylgruppe darstellt, von deren Enantiomeren, Diastereoisomeren und Epimeren sowie von deren Additionssalzen mit einer pharmazeutisch annehmbaren Säure.

3. Verfahren nach Anspruch 1 zur Herstellung von Verbindungen der Formel (I), worin R₁ und R₂ gleichzeitig Wasserstoffatome bedeuten, von deren Enantiomeren, Diastereoisomeren und Epimeren sowie von deren Additionssalzen mit einer pharmazeutisch annehmbaren Säure.

4. Verfahren nach einem der Ansprüche 1, 2 oder 3 zur Herstellung von 4-(2-Cyclopentyl-3,3,3-trifluorpropan-1-yl)-imidazol, von dessen Enantiomeren sowie von dessen Additionssalzen mit einer pharmazeutisch annehmbaren Säure.

5. Verfahren nach einem der Ansprüche 1 oder 3 zur Herstellung von 4-(2-Phenyl-3,3,3-trifluorpropan-1-yl)-imidazol und von dessen Enantiomeren sowie von dessen Additionssalzen mit einer pharmazeutisch annehmbaren Säure.

6. Verfahren nach Anspruch 1 zur Herstellung von Verbindungen der Formel (VIII), die für die Herstellung der Verbindungen der Formel (I) nützlich sind.

7. Verfahren nach Anspruch 1 zur Herstellung von Verbindungen der Formel (IX), die für die Herstellung der Verbindungen der Formel (I) nützlich sind.

8. Verfahren nach Anspruch 1 zur Herstellung von Verbindungen der Formel (X), die für die Herstellung der Verbindungen der Formel (I) nützlich sind.
